# FASCICULE DE BREVET EUROPEEN

(11) **EP 0 507 825 B1**
(45) Date de publication et mention de la délivrance du brevet: **07.08.1996**
(21) Numéro de dépôt: 91901812.7
(22) Date de dépôt: 27.12.1990
(51) Int. Cl.: A61K 38/00

(54) **COMPOSITION ANTITUMORALE A BASE DE POLYPEPTIDES AYANT L'ACTIVITE DE L'INTERLEUKINE 2 HUMAINE**
ANTITUMORZUSAMMENSETZUNG DIE POLYPEPTIDE MIT DER AKTIVITÄT DES HUMANEN INTERLEUKIN-2 ENTHÄLT
ANTITUMORAL COMPOSITION BASED ON POLYPEPTIDES HAVING HUMAN INTERLEUKIN 2 ACTIVITY

(30) Priorité: 30.10.1990 FR 9013441
(43) Date de publication de la demande: 14.10.1992
(73) Titulaire: ROUSSEL UCLAF, F-93230 Romainville (FR)
(72) Inventeur: TURSZ, Thomas, F-94805 Villejuif Cédex (FR); BRANDELY, Maud, F-75017 Paris (FR)
(74) Mandataire: Vieillefosse, Jean-Claude
(86) Numéro de dépôt international: FR9000951
(87) Numéro de publication internationale: WO9207577

(56) Documents cités:
- EP-A- 0 353 150
- Biological Abstracts, vol. 89, no. 6, 15 mars 1990, Philadelphia, PA, US; Mow-Ming Hsu et al.: "Response to and concentration of interleukin-2 in patients with nasopharyngeal carcinoma", see page AB-623, abstract no. 60857
- Journal of Biological Response Modifiers, vol. 6, no. 4, 1987, Raven Press, New York, US; J.E. Kolitz et al.: "Expansion of activated T-lymphocytes in patients treated with recombinant interleukin 2", pp. 412-429

## Description

La présente invention concerne l'utilisation de l'interleukine 2 humaine pour la préparation de compositions pharmaceutiques destinées au traitement de tumeurs malignes épithéliales des voies aérodigestives supérieures ou du thymus par voie intraveineuse en perfusion continue de 24 heures.

L'Interleukine 2 (IL₂) qui est une lymphokine produite par les lymphocytes T activés possède une activité immunomodulatrice et une activité antitumorale décrites par exemple par Fletcher, M et al. (Lymphokine Research 6 1987 47-57), activités qui comprennent en particulier la capacité d'initier la prolifération des lymphocytes T et l'induction de la cytotoxicité des cellules NK (natural killer) et des cellules LAK (lymphokine activated killer). On a observé que l'administration de l'IL₂ soit seule à haute dose soit associée avec des cellules LAK est capable d'induire la régression de certains cancers établis chez la souris et chez des patients ayant des cancers métastatiques tel que le mélanome, le cancer du rein, le cancer colorectal ou le lymphome non Hodgkinien (Rosenberg, S A et al. N. Engl. J. Med. 1987 316 889-897).

Les tumeurs malignes épithéliales, ou épithéliomes, qui sont des tumeurs dues à une prolifération néoplasique des cellules épithéliales soit de l'épiderme, soit des muqueuses épidermoides, sont histologiquement diagnostiquées, en particulier dans les voies aérodigestives supérieures ou dans le thymus.

Les épithéliomes des voies aérodigestives supérieures concernent les différents sites du pharynx, parmi lesquels le nasopharynx qui représente une localisation relativement fréquente avec différents sous types histologiques, le plus souvent de type lymphoépithélial caractérisé par un épithéliome indifférencié infiltré de lymphocytes.

Le traitement majeur des épithéliomes est la radiothérapie, généralement précédée d'une polychimiothérapie ou associée avec celle-ci. Le pronostic variable selon le stade d'évolution de l'épithéliome est mauvais dans la mesure où un pourcentage important des malades traités développe ensuite des métastases. Ainsi une étude portant sur 49 patients atteints de carcinomes du nasopharynx et traités par radiothérapie soit seule, soit précédée d'une chimiothérapie, décrit un taux moyen de survie de 42 % à 5 ans (Stein, M et al. J. Surg. Oncol. 1988 37 (2) 84-88). Malgré un pronostic gui semble meilleur lorsgu'il s'agit de lymphoépithéliomes, un faible taux de survie de seulement 13 % est observé aprés 5 ans dans une étude portant sur 150 malades atteints de carcinome nasopharyngé le plus fréquemment de type lymphoépithélial, bien qu'un taux de rémission complète d'environ 65 % ait été décrit aprés une radiothérapie précédée ou non d'une chimiothérapie (Koppibar, S, B et al. J. Surg. Oncol. 1988 39 (3) 179-182). L'absence de traitement efficace est également reconnu, notamment chez 29 enfants, tous atteints de lymphoépithéliomes du nasopharynx parmi lesquels plus de 50 % développent des métastases secondaires dans un délai de 2 ans suivant l'arrêt de la radiothérapie (Pao, W, J et al. In. J. Radiat. Oncol. Biol. Phys 1989 17 (2) 299-305).

Les thymomes malins qui sont des tumeurs du thymus dues à une prolifération néoplasique des cellules épithéliales du thymus comprennent essentiellement les thymomes malins envahissants sans atypie, parmi lesquels peut être éventuellement distingué le sous-type lymphoépithélial ou lymphocytaire selon la population cellulaire qui les accompagne, et les carcinomes atypiques du thymus.

Contrairement aux thymomes encapsulés bénins pour lesquels existent des traitements appropriés tels que la chirurgie, la radiothérapie seules ou associées, les thymomes malins, en particulier les thymomes malins envahissants sans atypie, sont peu sensibles aux traitements conventionnels. De plus, leur fréquence d'apparition augmente (23 à 66 % de l'ensemble des thymomes).

Ainsi les thymomes malins envahissants traités soit par chirurgie, soit par radiothérapie comme premier traitement, suivi éventuellement par une chimiothérapie, ont un mauvais pronostic : jusqu'à 30 % des malades développent secondairement des métastases. Les résultats décrits chez des malades atteints de thymomes lymphoépithéliaux (Arriagada, R et al. Eur J Cancer Clin Oncol 20 (n° 1) 69-74 1984) ou chez des malades atteints de thymomes lymphoépithéliaux ou lymphocytaires (Goldel, N et al. Cancer 63 1493-1500 April 15 1989) sont décevants à l'exception de quelques résultats encourageants décrits après traitement par polychimiothérapie de thymomes malins envahissants riches en cellules épithéliales indifférenciées (Dy, C et al. Journal of Clinical Oncology 6 (n° 3) 536-542 March 1988). Il n'existe donc pas de traitement reconnu, en particulier dans les formes envahissantes des thymomes malins ou dans les carcinomes atypiques du thymus pour lesquels on observe des taux élevés de cas résistants, en particulier à la chimiothérapie.

La réponse à l'IL2 de malades ayant un carcinome nasopharyngé a été étudiée par mesure des cellules mononucléaires et de la concentration en IL₂ (Hsu et al. Biological Abstracts, 89, (6), AB-623, n° 60857).

Aucun résultat clinique n'a décrit l'utilisation de l'IL₂ dans le traitement de tumeurs épithéliales des voies aérodigestives supérieures.

Un résultat clinique a montré l'inefficacité de la thérapie dans les thymomes malins avec l' IL₂ seule, notamment avec une mutéine de l'IL₂ chez un patient atteint de thymome ayant reçu une dose de 1x 10⁶ U/M² par voie intraveineuse en perfusion de 6 heures selon un cycle constitué de 2 fois 5 jours (Kolitz, J E et al. Journal of Biological Response Modifiers 6 412-429 1987).

Or de façon surprenante, la demanderesse vient d'obtenir des résultats montrant que l'IL₂ seule présente une activité sur les épithéliomes.

L'invention concerne donc l'utilisation de l'interleukine 2 humaine pour préparer une composition pharmaceutique destinée au traitement des tumeurs malignes épithéliales des voies aérodigestives supérieures ou du thymus par voie intraveineuse en perfusion continue de 24 heures.

On entend par l'IL₂ humaine l'IL₂ humaine naturelle, l'IL₂ humaine recombinante c'est à dire obtenue par la technologie de l'ADN recombinant, par exemple comme cela est décrit par Taniguchi, T et al. Nature 1983 302 305-310 ou dans le brevet EP 91530 B, des allèles ou des dérivés de ces produits tels que décrits par exemple par Ju, C et al. J. Biol. Chem. 1987 262 5723-5731.

Les tumeurs des voies aérodigestives supérieures que concerne l'invention comprennent les épithéliomes du pharynx en général, par exemple les épithéliomes du nasopharynx, des amygdales ou de l'oesophage. Les tumeurs du thymus concernées par l'invention comprennent le thymome malin envahissant épithélial, lymphoépithélial ou lymphocytaire et les carcinomes du thymus.

L'invention a notamment pour objet, l'utilisation caractérisée en ce que la tumeur des voies aérodigestives supérieures est un lymphoépithéliome, diagnostiqué histologiquement.

L'invention a également pour objet l'utilisation caractérisée en ce que la tumeur du thymus est un thymome malin envahissant lymphoépithélial, diagnostiqué par les examens histologique, cytologique et biologique usuels. L'utilisation selon l'invention est destinée au traitement du thymome malin, au traitement de la rechute de celui-ci ou au traitement préventif de celle-ci.

L'utilisation de l'IL₂ selon l'invention est illustrée par une réponse partielle chez un malade atteint de thymome malin ayant subi sans succés un traitement antérieur de chimiothérapie.

L'invention a particulièrement pour objet l'utilisation caractérisée en ce que l'IL₂ humaine est une IL₂ recombinante pure.

Les compositions pharmaceutiques préparées selon l'invention renferment une IL₂ humaine recombinante, des allèles ou des dérivés de celle-ci, telle que décrite ci-dessus, pour laquelle on utilise des techniques de purification connues de l'homme de métier, qui permettent de préparer des produits purs.

L'invention a plus particulièrement pour objet l'utilisation caractérisée en ce que l'IL₂ est une IL₂ recombinante non glycosylée sous forme réduite. L'IL₂ non glycosylée utilisée est notamment celle ayant la séquence de l'IL₂ naturelle à 133 aminoacides avec éventuellement une méthionine N-terminale supplémentaire, dont les 3 cystéines en position 58, 105 et 125 sont sous la forme réduite, manifestant une activité biologique comparable à celle de l'IL₂ oxydée ayant la même séquence comportant un pont disulfure en position 58-105 et qui est décrite dans la demande de brevet européen EP 0353150. Par forme réduite, on entend que les restes cystéines que contient l'IL₂ renferment un groupement sulfhydryle libre dont la détermination est faite, par exemple, par spectrophotométrie avec la dithiodipyridine comme réactif des thiols. L'activité biologique est déterminée par mesure de la prolifération de lignées cellulaires leucémiques de souris dépendantes de l'IL₂ CTLL-2, avec un test colorimétrique au sel de tétrazolium (Mossmann, T J. Immunol. Meth 1983 65 55-63). L'activité spécifique des IL₂ recombinantes utilisées dans l'invention est au moins égale à 0.5 .10⁷ U BRMP/mg, de préférence 1.10⁷ U BRMP/mg. L'unité d'activité IL₂ est définie comme la quantité qui produit 50 % de la réponse maximum dans le test. On utilise comme standard un échantillon "Biological Response Modifier Programm (BRMP) reference agent human IL₂ "(jurkat)" fourni par National Cancer Institute (NCI).

L'invention a spécialement pour objet l'utilisation caractérisée en ce que l'IL₂ est administrée par voie intraveineuse en perfusion continue à la dose de 2 à 25.10⁶ U/M² par jour et plus spécialement celle caractérisée en ce que l'IL₂ est administrée à la dose de 20.10⁶ U/M² par jour.

L'invention a tout spécialement pour objet l'utilisation caractérisée en ce que l'IL₂ est administrée par cycle de 3 à 5 jours consécutifs et caractérisée en ce que l'IL₂ est administrée de façon répétée pendant au moins 3 cycles non consécutifs.

La dose administrée, la fréquence de l'injection et la durée du traitement varient en fonction de l'état du malade.

L'IL₂ est comprise dans une composition pharmaceutique, de préférence lyophilisée en flacon-dose contenant de 0.05 à 2 mg de principe actif et que l'on reconstitue avec de l'eau distillée pour injection. La solution obtenue est immédiatement diluée à l'aide d'un soluté, par exemple le glucose à 5 % pour l'administration en perfusion intraveineuse.

Selon l'utilisation préférée de l'invention, l'IL₂ est l'IL₂ recombinante réduite ci-dessus dont un exemple de préparation pharmaceutique est donnée plus loin, la dose est de 20.10⁶ U/M² par jour, selon un cycle de 3 à 5 jours consécutifs, la durée de l'administration est de 3 cycles non consécutifs en perfusion continue par voie intraveineuse.

Toutes les publications qui sont citées sont incorporées pour référence, dans le texte de la présente demande.

Les exemples suivants illustrent l'invention sans toutefois la limiter :

### EXEMPLE 1 : composition pharmaceutique pour perfusion.

On a réalisé une préparation pour injection par voie intraveineuse en perfusion de formule :

| | |
|---|---|
| IL₂ réduite | 0.5 mg |
| acide citrique | 5 mg |
| mannitol | 50 mg |
| eau stérile | 1 ml |
| glucose à 5 % | 50 ml |

### EXEMPLE 2 : étude clinique dans le traitement d'un thymome malin.

L'étude inclut un malade ayant un thymome malin lymphoépithélial histologiquement confirmé envahissant le mediastin, le poumon droit, la plévre, le péricarde et le diaphragme, ayant reçu un traitement de première ligne par 2 cycles de polychimiothérapie conventionnelle comprenant du cisplatine, de l'endoxan et de la vindesine puis un traitement de deuxième ligne par l'adriamycine à forte dose (100 mg/M²) sans qu'ait été observée une régression de la tumeur.

L'absence, sous chimiothérapie, de régression des masses médiastinales et pleuropulmonaires visualisées par la radiographie et le scanner thoracique, a incité à débuter un traitement par l'IL₂, 4 mois et demi après la fin de cette chimiothérapie.

Les compositions d'IL₂ préparées selon l'invention permettent d'injecter en perfusion continue par voie intraveineuse une dose de 20 x 10⁶ U/M² par jour pendant 3 à 5 jours consécutifs selon un premier cycle de 5 jours, un deuxième cycle de 4 jours et un troisième cycle de 3 jours avec un intervalle de 9 jours entre chaque cycle. Les lésions tumorales du malade sont évaluées avant et à la fin de chaque cycle par radiographie puis par scanographie. On utilise les compositions décrites à l'exemple 1.

Le traitement par l'IL₂ a entrainé une réponse partielle au niveau des masses pleurales qui ont diminuées de plus de 75 %, observée par radiographie et confirmée par scanner thoracique, sans modification de la masse thymique.Une thoracotomie est pratiquée au décours du traitement par l'IL₂ pour vérifier l'état de la loge thymique.La masse tumorale résiduelle peut être totalement enlevée par le chirurgien et se révèle massivement nécrosée, alors que l'exérése complète des métastases résiduelles au niveau pulmonaire et diaphragmatique n'est techniquement pas réalisable.

## Revendications

1. Utilisation de l'interleukine 2 humaine pour préparer une composition pharmaceutique destinée au traitement des tumeurs malignes épithéliales des voies aérodigestives supérieures ou du thymus par voie intraveineuse en perfusion continue de 24 heures.

2. Utilisation selon la revendication 1 caractérisée en ce que la tumeur des voies aérodigestives supérieures est un lymphoépithéliome.

3. Utilisation selon la revendication 1 caractérisée en ce que la tumeur du thymus est un thymome malin envahissant lymphoépithélial.

4. Utilisation selon l'une des revendications 1 à 3 caractérisée en ce que l'IL₂ humaine est une IL₂ recombinante pure.

5. Utilisation selon la revendication 4 caractérisée en ce que l'IL₂ est une IL₂ recombinante non glycosylée sous forme réduite.

6. Utilisation selon la revendication 5 caractérisée en ce que l'IL₂ est administrée par voie intraveineuse en perfusion continue à la dose de 2 à 25.10⁶ U/M² par jour.

7. Utilisation selon la revendication 6 caractérisée en ce que l'IL₂ est administrée à la dose de 20.10⁶ U/M² par jour.

8. Utilisation selon la revendication 7 caractérisée en ce que l'IL₂ est administrée par cycle de 3 à 5 jours consécutifs.

9. Utilisation selon la revendication 8 caractérisée en ce que l'IL₂ est administrée de façon répétée pendant au moins 3 cycles non consécutifs.

## Claims

1. Use of human interleukin 2 for preparing a pharmaceutical composition intended for the treatment of malignant epithelial tumours of the upper aerodigestive tracts or of the thymus by intravenous route as a continuous 24-hour perfusion.

2. Use according to claim 1, characterized in that the tumour of the upper aerodigestive tracts is a lymphoepithelioma.

3. Use according to claim 1, characterized in that the tumour of the thymus is an invasive lymphoepithelial malignant thymoma.

4. Use according to one of claims 1 to 3, characterized in that the human IL₂ is a pure recombinant IL₂.

5. Use according to claim 4, characterized in that the IL₂ is a non-glycosylated recombinant IL₂ in reduced form.

6. Use according to claim 5, characterized in that the IL₂ is administered by intravenous route as a continuous perfusion at a dose of 2 to 25x10⁶ U/M² per day.

7. Use according to claim 6, characterized in that the IL₂ is administered at a dose of 20x10⁶ U/M² per day.

8. Use according to claim 7, characterized in that the IL₂ is administered in a cycle of 3 to 5 consecutive days.

9. Use according to claim 8, characterized in that the IL₂ is administered in a repeated fashion for at least 3 non-consecutive cycles.

## Patentansprüche

1. Verwendung von menschlichem Interleukin 2 zur Herstellung einer pharmazeutischen Zusammensetzung zur Behandlung von malignen Epitheltumoren der oberen Atmungs-Verdauungs-Wege oder des Thymus auf intravenösem Weg durch kontinuierliche 24stündige Infusion.

2. Verwendung nach Anspruch 1, dadurch **gekennzeichnet,** daß der Tumor der oberen Atmungs-Verdauungs-Wege ein Lymphepitheliom ist.

3. Verwendung nach Anspruch 1, dadurch **gekennzeichnet,** daß der Tumor des Thymus ein malignes lymphepithelial-eindringendes Thymom ist.

4. Verwendung nach einem der Ansprüche 1 bis 3, dadurch **gekennzeichnet,** daß das menschliche IL-2 ein reines rekombinantes IL-2 ist.

5. Verwendung nach Anspruch 4, dadurch **gekennzeichnet,** daß das IL-2 ein rekombinantes nichtglykosyliertes IL-2 in reduzierter Form ist.

6. Verwendung nach Anspruch 5, dadurch **gekennzeichnet,** daß das IL-2 auf intravenösem Weg durch kontinuierliche Infusion in einer Dosis von 2 bis 25.10⁶ E/m² pro Tag verabreicht wird.

7. Verwendung nach Anspruch 6, dadurch **gekennzeichnet,** daß das IL-2 in einer Dosis von 20.10⁶ E/m² pro Tag verabreicht wird.

8. Verwendung nach Anspruch 7, dadurch **gekennzeichnet,** daß das IL-2 in Zyklen von 3 bis 5 aufeinanderfolgenden Tagen verabreicht wird.

9. Verwendung nach Anspruch 8, dadurch **gekennzeichnet,** daß das IL-2 wiederholt über mindestens drei nichtaufeinanderfolgende Zyklen verabreicht wird.
